# EUROPEAN PATENT APPLICATION

(11) **EP 4 516 303 A1**
(43) Date of publication of application: **05.03.2025**
(21) Application number: 24175038.9
(22) Date of filing: 09.05.2024
(51) Int. Cl.: A61K 31/7034, A61K 31/704, A61K 31/047, A61K 31/519, A61K 36/27, A61K 36/53, A61P 5/26

(54) **COMPOSITION INCLUDING TEUPOLIOSIDE AND INOSITOL**

(30) Priority: 30.08.2023 IT 202300017805
(71) Applicant: IDI Integratori Dietetici Italiani S.r.l., 95020 Aci Bonaccorsi (CT) (IT)
(72) Inventor: BOTTINO, Alessandro, 95020 Aci Bonaccorsi CT (IT); BOTTINO, Roberto, 95020 Aci Bonaccorsi CT (IT)
(74) Representative: Di Giovine, Paolo

(57) **Abstract**

The present invention relates to a composition comprising teupolioside and inositol or a stereoisomer thereof, and to the use thereof in the prevention, treatment or in aiding the treatment of pathologies due to hyperandrogenism.

The present invention then relates to said composition, as well as to a pharmaceutical composition, homeopathic product, a phytotherapeutic drug, cosmetics, food supplement, food product, beverage, a medical device, a medicated food, food for special medical purposes and a kit of parts comprising the composition, and the use thereof in the prevention, treatment or in aiding the treatment of pathologies due to hyperandrogenism.

## Description

### FIELD OF THE INVENTION

The present invention relates to a composition comprising teupolioside and inositol or a stereoisomer thereof, and the use thereof in the prevention, treatment or in aiding the treatment of pathologies due to hyperandrogenism.

The present invention then relates to said composition, as well as to a pharmaceutical composition, a homeopathic product, a phytotherapeutic drug, cosmetics, nutraceutical, food supplement, a medical device, medicated food, food for special medical purposes, food product, a beverage and a kit of parts comprising the composition, and the use thereof in the prevention, treatment or in aiding the treatment of pathologies due to hyperandrogenism.

### STATE OF ART

Hyperandrogenism is a condition characterized by high levels of androgenic hormones in the body which can cause a series of unwished disorders, such as hirsutism (excessive hair growth), alopecia (hair loss) and PCOS (polycystic ovary syndrome). These conditions can affect negatively the quality of life of people suffering therefrom. Hirsutism affects from 5% to 15% of women of reproductive age, with about 80% of these women having polycystic ovary syndrome (PCOS). The etiopathogenesis of PCOS remains unclear, the clinical features of PCOS include hyperandrogenism, generally appeared as hirsutism and acne, and both these clinical symptoms are treated with oral contraceptive pills (OCP), topical or antiandrogen drugs.

Among the most used drugs there are oral contraceptives containing estrogens and progestins, commonly prescribed to treat PCOS and hirsutism, and they act by adjusting sex hormones and by reducing the production of androgens; antiandrogens, spironolactone and flutamide, which lock the effects of androgens in the body and are often used to treat hirsutism and androgenetic alopecia in women; metformin, a drug commonly prescribed for type 2 diabetes, sometimes used even for PCOS treatment since it is believed to help reducing insulin resistance, one of the factors associated to PCOS.

Apart from the above-mentioned drugs, in recent years there has been a growing interest in the use of inositol in the treatment of hyperandrogenism and its clinical manifestations. Inositols are chemical compounds called carbocyclic polyols, present in most life forms. There are several stereoisomers of inositol, thereamong myoinositol (Ml) and d-chiroinositol inosol (DCI), which were studied for their potential beneficial effects on hormone metabolism and blood sugar regulation.

Some preliminary studies suggest that taking inositol, in particular MI and DCI, could contribute to improve insulin resistance, to regulate the period and to reduce the symptoms of hirsutism and of alopecia in women with PCOS. However, the need remains to provide a composition of bioactive substances with multitarget activity effective for treating conditions associated to hyperandrogenism.

### SUMMARY OF THE INVENTION

The authors of the present invention have surprisingly found that a composition comprising a new combination of active ingredients, in particular teupolioside and inositol or a stereoisomer thereof, allows to treat effectively conditions associated to hyperandrogenism.

In particular, in the treatment of pathologies such as hirsutism, alopecia and especially in the treatment of polycystic ovary syndrome (PCOS).

The herein described invention has the following advantages:
- Multitarget approach for the treatment of PCOS. The new invention, differently from the treatments currently available for PCOS, acts on two different pathways involved in the onset of the symptoms related to hyperandrogenism. The involved action mechanisms are different and they act at two different levels, inositol acts by exploiting its own insulin-sensitizing action and teupolide, by modulating the activity of 5-alpha-reductase through oxidation of coenzyme NADPH
- Enhanced effect of the two ingredients with blocking of the biochemical processes responsible for the androgenic signs typical of the pathology
- Use of "food grade" ingredients, characterized by a high safety level and without the adverse reactions typical of the drugs
- High compliance of the preparation, with consequent reduction in patient dropout.

Objects of the present invention are:
a composition comprising teupolioside and inositol or a stereoisomer thereof and the use thereof in the prevention, treatment or in aiding the treatment of pathologies due to hyperandrogenism
food supplement or food product or a beverage or medicated food or food for special medical purposes, medical device or cosmetic product or pharmaceutical composition or a homeopathic product or a phytotherapeutic drug or nutraceutical composition comprising or consisting of the composition according to any one of the herein described embodiments;
a kit of parts for use in the prevention, treatment or in aiding the treatment of pathologies due to hyperandrogenism, comprising teupolioside and inositol or a stereoisomer thereof in a dosage form for simultaneous, separate, or sequential administration thereof. Other advantages and features of the present invention will result evident from the detailed description.

### GLOSSARY

In the present invention, under "Teupolioside" reference is made to the compound also known as Lamiuside "A", a phenylpropanoid glycoside deriving from *Ajuga reptans,* a plant used in the Mediterranean traditional herbal medicine used for several diseases, thereamong cardiovascular complications, skin disorders, respiratory tract disorders. The teupolioside compound has the following chemical structure formula:

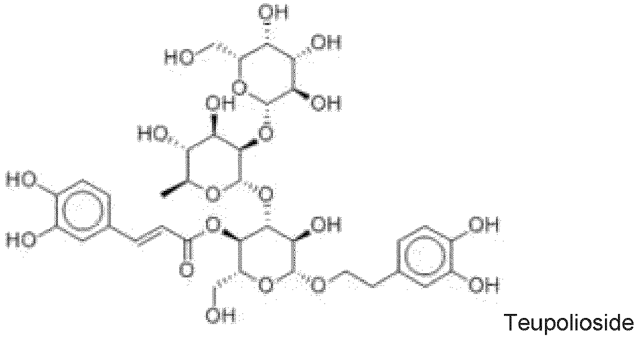

In the present invention, under "inositol or a stereoisomer thereof" reference is made to a chemical substance which can be inositol or one of the structural variants thereof sharing the same chemical formula but differentiating in the spatial arrangement of atoms. Inositol is a carbocyclic polyol synthetized endogenously starting from glucose 6-phosphate and it is involved in a series of biological processes in the human body. Inositol stereoisomers are optically active forms of the molecule which can differ in the three-dimensional arrangement of the functional groups. The specific selection of inositol or a stereoisomer thereof in the present invention can depend upon its specific biological activity, upon the wished bioavailability or other features of interest in the context of the application of the present invention. The structure formulas of inositol and stereoisomers thereof are reported hereinafter:

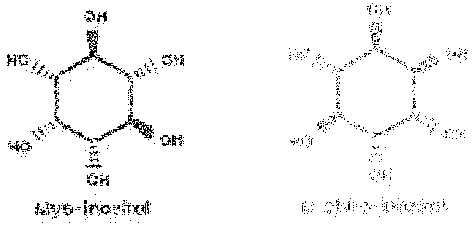

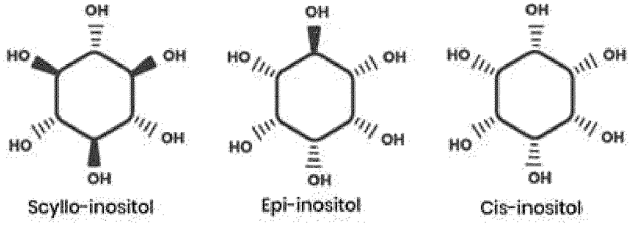

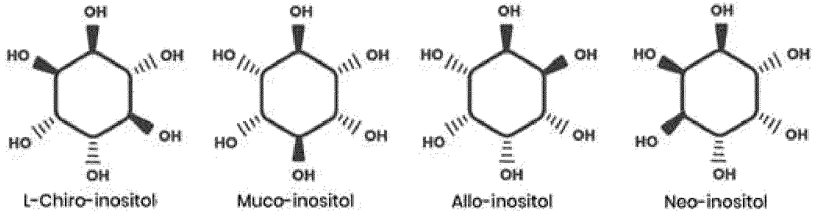

In the present invention, under the term "micronized form" reference is made to a particular form of a substance which was subjected to a micronization process. Micronization is a technique for reducing the sizes of particles of a substance so as to obtain very small particles, in the order of micrometers. According to an embodiment micronization of inositol can be implemented according to the process described in the patent number 102019000025063 granted on 10 December 2021.

In the present invention, under the term "food supplement" a food product is meant intended to integrate the common diet and which constitutes a concentrated source of nutrients, such as vitamins and mineral salts, or other substances with nutritional or physiological effects, in form of unit dose (compare also Directive 2002/46/EC of 10 June 2002 and Legislative Decree Nr. 169 Art.2 of 21 May 2004).

### DETAILED DESCRIPTION

The present invention relates to a composition comprising teupolioside and inositol or a stereoisomer thereof, the composition is particularly effective in the treatment of conditions associated to hyperandrogenism. According to an embodiment said stereoisomer of inositol is at least one selected from myoinositol, epiinositol, d-chiroinositol, d-Chiro- inositol, Scyllo-inositol, Cis- inositol, Mucoinositol, Neo- inositol, Allo-inositol, Epi- inositol or a mixture thereof.

In a preferred embodiment, said teupolioside is present in a concentration from 0.001% to 0.2% w/w, preferably from 0.0075% to 0.02% w/w.

In an embodiment, said teupolioside is in the form of *Ajuga reptans* extract.

According to an embodiment said *Ajuga reptans* extract is titrated between 5 and 50% in teupolioside, preferably between 10 and 25% in teupolioside, wherein the percentage designates the percentage by weight of teupolioside with respect to the weight of the extract.

In a preferred embodiment, said inositol or a stereoisomer thereof is present in a concentration from 0.01% to 10% w/w, preferably from 0.5% to 4% w/w.

In an embodiment, the composition of the invention comprises at least one further active ingredient selected from gymnema sylvestre extract and/or L-5 calcium methyltetrahydrofolate.

In a preferred embodiment, the composition of the invention further comprises an extract of gymnema sylvestre and L-5 calcium methyltetrahydrofolate.

In an embodiment, said *gymnema sylvestre* is present in a concentration from 0.001% to 1% w/w, preferably from 0.1% to 0.5% w/w.

In an embodiment, said L-5 calcium methyltetrahydrofolate is present in a concentration from 0.00001 a 0.02 Y% w/w, preferably from 0.0002 to 0.01 w/w.

In an additional embodiment, said *gymnema sylvestre* extract and/or said inositol or a stereoisomer thereof is in micronized form, preferably between 100 and 500 micron.

In an embodiment, the composition of the invention is formulated for oral, rectal, nasal, aerosol, sublingual, systemic, topical administration.

In an embodiment, the composition of the invention is in the form of tablet, hard capsule or softgel, extended-release capsule, suspension, emulsion, granulate, drops, syrup, elixir, pomade, ointment, suppository, cream, gel, spray, powder or solution.

The present invention further relates to food supplement or food product or beverage or medical device or cosmetic product or medicated food or food for special medical purposes or pharmaceutical composition or homeopathic product or phytotherapeutic drug or nutraceutical composition comprising or consisting of the composition according to any one of the herein described embodiments and at least one pharmaceutically acceptable food or cosmetic excipient and/or carrier.

The herein described compositions could include one or more excipients and/or diluents, the person skilled in the art could select such excipients and/or diluents suitable depending upon the selected formulation.

Moreover, each one of these forms can be of conventional type or with modified release (for example with immediate, rapid, prolonged or delayed release).

Under the expression "pharmaceutical forms with modified release (or not conventional forms)" the dosage forms are herein meant, therefor the profile for releasing (and then absorbing) the active ingredient depends upon both the chemical and physical features of such ingredient and upon the characteristic technology of the formulation. The factor determining the absorption profile is the speed of releasing the active ingredient from the pharmaceutical form.

Under the expression "agent which modifies the release" of an active ingredient in the organism a compound is herein meant capable of modifying the speed or the dissolution site and, consequently, the absorption of an active ingredient by the tissues and the body fluids, therewith it comes in contact after administration.

Examples of various classes of such agents are: disintegrating agents (compounds which facilitate the disintegration of solid pharmaceutical forms, by increasing the surface in contact with the biological fluids and thus by increasing the speed for releasing and absorbing the active ingredient); surfactants (compounds which are added to the solid preparations to increase the product wettability and then the disintegration thereof); polymers (compounds which modify the time or the site for releasing the active ingredient, for example in preparations with prolonged release or gastro-resistant preparations).

All these above-listed agents can be used for mono-or multi-layer tablets, swallable, chewable, buccal or sublingual tablets or granules, for capsules or sachets.

Under the term "excipient", reference is made to conventional excipients, that is compounds that are inert towards the active ingredient and the pharmaceutical form. Examples of different classes of these ingredients are: diluents (compounds added when the mass of the active ingredient is not sufficient for preparing the composition); lubricants (preventing the power from adhering to the mechanical parts during the manufacturing process); aggregating agents (compounds which increase the cohesion of powders); dyes (used to improve the presentation of some pharmaceutical dosage forms, for example capsules, or to classify them based upon the therapeutical category to which they belong or to distinguish them from other similar products); sweeteners or flavourings (added to improve the organoleptic features of the products); or antioxidants-antimicrobials (used to prolong the product shelf life).

Examples of such diluting, aggregating or binding agents, lubricants, slippers, disaggregating agents, solubilizers and/or pH regulators are the following ones: light magnesium oxide, magnesium hydroxide, alginic acid, stearic acid, hydrogenated vegetable oils (palm, oleic or behenic), cocoa butter, cocoa paste, chitosan, yeast, sodium carboxymethylcellulose (CMC), pregelatinized corn starch.

The most commonly used conventional excipients are the following ones: lactose, glucose, sucrose, mannite (or mannitol), kaolin, talc, bentonite, titanium dioxide, xylitol, maltitol, sorbitol, sucralose, acesulfame K, aspartame, neohesperidin, fructose, dextrose, maltose, "spraydried" malt, sodium aspartate, maltodextrin, sodium chloride, hydroxypropyl methylcellulose, erythritol, citrus extract, silica gel, vegetable fibres (such as pea fibre), flavours and aroma, such as for example mint flavour (peppermint, crispa, sweet), badiana anethole, vanilla, sage, grapefruit, peach, orange, lemon or lime, sodium glutamate an fish meal.

In an embodiment, said food supplement, medical device, medicated food, food product, beverage, food for special medical purposes, cosmetic product, pharmaceutical composition, homeopathic product, phytotherapeutic drug, nutraceutical composition, or the composition of the invention according to any one of the herein described embodiments, is for use as medicament or coadiuvant to a therapeutic treatment.

In an embodiment, the composition of the invention and said food supplement, medical device, medicated food, food for special medical purposes or cosmetic product, nutraceutical composition or pharmaceutical composition according to any one of the herein described embodiments, is for use in the prevention, treatment or in aiding the treatment of pathologies due to hyperandrogenism.

In an embodiment, said pathologies are selected from Polycystic ovary syndrome (PCOS), hirsutism, alopecia, metabolic syndrome, amenorrhea, oligoamenorrhea, acne and infertility.

The present invention also relates to a kit of parts for use in the prevention, treatment or in aiding the treatment of pathologies due to hyperandrogenism, comprising teupolioside and inositol or a stereoisomer thereof in a dosage form for simultaneous, separate, or sequential administration thereof. In particular, said pathologies are selected from Polycystic ovary syndrome (PCOS), hirsutism, alopecia, metabolic syndrome, amenorrhea, oligoamenorrhea, acne and infertility.

The present invention also relates to the association of teupolioside and inositol or a stereoisomer thereof for use in the treatment of pathologies due to hyperandrogenism, wherein said pathologies are preferably selected from Polycystic ovary syndrome (PCOS), hirsutism, alopecia, metabolic syndrome, amenorrhea, oligoamenorrhea, acne and infertility.

In any part of the present description and claims, the term comprising can be replaced by the term "consisting of".

Examples are reported herebelow having the purpose of illustrating better the methods disclosed in the present description, such examples are in no way to be considered a limitation of the preceding description and of the subsequent claims.

### EXAMPLES

Formulation example 1:
Miolnositol 500 mg
Teupolioside 7.5 mg
Gymnema 100 mg
L-5 calcium methyltetrahydrofolate 200 µg
+ excipients

Formulation example 2:
D-chiroinositol 250 mg
Teupolioside 7.5 mg
Gymnema 100 mg
L-5 calcium methyltetrahydrofolate 200 µg.
+ excipients

Formulation example 3:
D-chiroinositol 500 mg
Ajuga reptans 30 mg, titrated at 10% or at 25% in teupolioside
Gymnema 100 mg
L-5 calcium methyltetrahydrofolate 200 µg.
+ excipients

Formulation example 4:
D-chiroinositol 500 mg
Ajuga reptans 75 mg, titrated at 10% in teupolioside
Gymnema 100 mg
L-5 calcium methyltetrahydrofolate 200 µg.
+ excipients

Formulation example 5:
Myi-inositol 500 mg
Ajuga reptans 30 mg, titrated at 25% in teupolioside
Gymnema 100 mg
L-5 calcium methyltetrahydrofolate 200 µg.
+ excipients

Formulation example 6:
D-chiroinositol 500 mg
Teupolioside 15 mg
Gymnema 200 mg
L-5 calcium methyltetrahydrofolate 400 µg
+ excipients

## Claims

1. A composition comprising teupolioside and inositol or a stereoisomer thereof.

2. The composition according to claim 1, wherein said stereoisomer of inositol is at least one selected from myoinositol, epiinositol, d-Chiro-inositol, Scyllo -inositol, Cis-inositol, Mucoinositol, Neo-inositol, Allo-inositol or a mixture thereof.

3. The composition according to any one of claims 1 or 2, wherein said teupolioside is in the form of *Ajuga reptans* extract, preferably said *Ajuga reptans* extract is titrated between 5 and 50% in teupolioside, preferably between 10 and 25% in teupolioside, wherein the percentages indicate the percentage by weight of teupolioside with respect to the weight of the extract.

4. The composition according to any one of the preceding claims, wherein said teupolioside is present in a concentration from 0.001% to 0.2% w/w, preferably from 0.0075% to 0.02% w/w, and/or said inositol or a stereoisomer thereof is present in a concentration from 0.01% to 10% w/w, preferably from 0.5% to 4% w/w.

5. The composition according to any one of the preceding claims, comprising at least one further active ingredient selected from gymnema sylvestre extract and/or L-5 calcium methyltetrahydrofolate, preferably said gymnema sylvestre extract is a leaf extract having a titre of gymnemic acids from 10% to 100%, even more preferably from 25% to 75% by weight with respect to the total extract.

6. The composition according to any one of the preceding claims for oral, rectal, nasal, aerosol, sublingual, systemic, topical administration.

7. The composition according to any one of the preceding claims in the form of a tablet, capsule, prolonged-release tablet, suspension, emulsion, granulate, drops, syrup, pomade, ointment, suppository, cream, gel, spray, powder or solution.

8. A food supplement or medical device or medicated food or food for special medical purposes or cosmetic product or pharmaceutical composition or nutraceutical composition or food product or beverage or homeopathic or phytotherapeutic product comprising or consisting of the composition according to any one of claims 1 to 7 and at least one pharmaceutically acceptable or food excipient and/or carrier.

9. The composition, food supplement, medical device, medicated food, food for special medical purposes, cosmetic product, nutraceutical composition or pharmaceutical composition for use according to claim 8, for use in the prevention, treatment or in aiding the treatment of pathologies due to hyperandrogenism.

10. The composition, food supplement, medical device, medicated food, food for special medical purposes, cosmetic product, nutraceutical composition or pharmaceutical composition for use according to claim 8 or 9, wherein said pathologies are selected from Polycystic ovary syndrome (PCOS), hirsutism, alopecia, metabolic syndrome, amenorrhea, oligoamenorrhea, acne and infertility.

11. A kit of parts for use in the prevention, treatment or in aiding the treatment of pathologies due to hyperandrogenism, comprising teupolioside and inositol or a stereoisomer thereof in a dosage form for simultaneous, separate or sequential administration thereof.
